# EUROPÄISCHE PATENTSCHRIFT

(11) **EP 3 630 326 B1**
(45) Veröffentlichungstag und Bekanntmachung des Hinweises auf die Patenterteilung: **20.11.2024**
(21) Anmeldenummer: 18740104.7
(22) Anmeldetag: 29.05.2018
(51) Int. Cl.: B01D 15/18

(54) **VERFAHREN ZUM TRENNEN VON NATURSTOFFGEMISCHEN MITTELS SCPC**
METHOD OF SEPARATING NATURAL MIXTURES USING SCPC
PROCÉDÉ DE SÉPARATION DE MÉLANGES DE MATIÈRES NATURELLES À L'AIDE DE SCPC

(30) Priorität: 29.05.2017 EP 17173304
(43) Veröffentlichungstag der Anmeldung: 08.04.2020
(73) Patentinhaber: Candoro ethics GmbH, 61381 Friedrichsdorf (DE)
(72) Erfinder: ENGLERT, Michael, 90478 Nürnberg (DE); RUTZ, Andreas, 91741 Dornhausen (DE)
(74) Vertreter: Becker Kurig & Partner Patentanwälte mbB
(86) Internationale Anmeldenummer: PCT/EP2018/064121
(87) Internationale Veröffentlichungsnummer: WO 2018/233991

(56) Entgegenhaltungen:
- EP-A1- 3 061 510
- ARNO HAZEKAMP ET AL: "Preparative Isolation of Cannabinoids from Cannabis sativa by Centrifugal Partition Chromatography", JOURNAL OF LIQUID CHROMATOGRAPHY & RELATED TECHNOLOGIES, vol. 27, no. 15, 11 January 2004 (2004-01-11), pages 2421 - 2439, XP055202081, ISSN: 1082-6076, DOI: 10.1081/JLC-200028170
- GOLL JOHANNES ET AL: "Study of the separation limits of continuous solid support free liquid-liquid chromatography: Separation of capsaicin and dihydrocapsaicin by centrifugal partition chromatography", JOURNAL OF CHROMATOGRAPHY A, ELSEVIER, AMSTERDAM, NL, vol. 1284, 8 February 2013 (2013-02-08), pages 59 - 68, XP029000702, ISSN: 0021-9673, DOI: 10.1016/J.CHROMA.2013.01.116
- PEDAN VASILISA ET AL: "Extraction of cocoa proanthocyanidins and their fractionation by sequential centrifugal partition chromatography and gel permeation chromatography", ANALYTICAL AND BIOANALYTICAL CHEMISTRY, SPRINGER, DE, vol. 408, no. 21, 18 June 2016 (2016-06-18), pages 5905 - 5914, XP036013845, ISSN: 1618-2642, [retrieved on 20160618], DOI: 10.1007/S00216-016-9705-7

## Beschreibung

Die Erfindung betrifft ein Verfahren zum Trennen und/oder Reinigen von einer oder mehr Cannabinoiden, aus einem Cannabispflanzenextrakt und deren Isolierung und Aufreinigung als auch Gewinnung mittels Sequential Centrifugal Partition Chromatography (sCPC) .

Der kontinuierliche TMB (True Moving Bed) Prozess als Gegenstromverfahren verbindet die Vorteile der Flüssig-Flüssig Chromatographie mit denen eines kontinuierlichen Prozesses, wie zum Beispiel dem SMB (Simulated Moving Bed) unter Einsatz einer Zentrifugalsäule bzw. rotierende Trennsäule. Bei der Flüssig-Flüssig Chromatographie wird sowohl mit flüssiger mobiler Phase, als auch mit flüssiger stationärer Phase gearbeitet und kein festes Säulenmaterial eingesetzt. Im Gegensatz zur SMB und deren verwandten Prozessen wird bei einer TMB die Bewegung der nicht-mischbaren Phasen nicht über eine Ventilschaltung erreicht, sondern über eine getaktete Phasenumkehr in einer ausgewählten Frequenz. Die grundlegende technische Lehre ist in WO 2005/011835 A1 offenbart.

Charakteristisch ist der kontinuierliche Wechsel der stationären Phase zur mobilen Phase und umgekehrt, d.h. es kann die dichtere oder weniger dichte Phase als mobile Phase ausgewählt werden und ein Wechsel dieser Auswahl ist während einer Trennung möglich. Daher wird diese präparative Chromatographie als "Sequential Centrifugal Partition Chromatography (sCPC)" bezeichnet. Die zu trennenden Stoffe A und B werden in einer Flüssig-Flüssig-Zentrifugalsäule aufgrund eines unterschiedlichen Verteilungskoeffizienten mit verschiedenen Geschwindigkeiten in den beiden Phasen verschiedener Dichte bewegt.

Eine sCPC-Apparatur weist mindestens einen Rotor mit vielen runden Metallplatten auf in denen sich beispielsweise über Eintausend in Serie verbundene Trennkammern (auch Rotorkammern genannt unter Ausbildung einer rotierenden Trennsäule) befinden. Mit Hilfe einer Pumpe wird im Rotor die stationäre flüssige Phase von einer mobilen flüssigen Phase(kontinuierlich) durchströmt. Der Rotor wird z.B. auf ca 1.000 U/min und mehr beschleunigt, so dass die Zentrifugalkraft die Trennung der beiden flüssigen Phasen aufgrund der Dichteunterschiede in den einzelnen Kammern bewirkt. Sobald die flüssige mobile Phase im Gleichgewicht (Equilibrium) mit der flüssigen stationären Phase liegt, kann die Probe bzw. das Substanzgemisch in den Rotor injiziert werden.

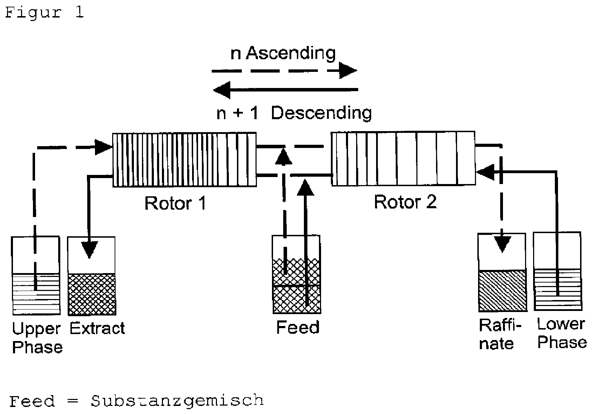

Die Stofftrennung erfolgt aufgrund verschiedener Adsorption in der mobilen bzw. stationäre Phase oder des jeweiligen Verteilungskoeffizienten (K) eines Stoffes zur mobilen/stationären Phase, wobei die Stoffe durch die einzelnen Trennkammern zum Rotorausgang bewegt und fraktioniert werden. Der charakteristische alternierende Wechsel von stationärer Phase und mobiler Phase erfolgt durch Steuerung der eingesetzten Pumpen mittels Ventilen in einer zu wählenden Frequenz. In einer zu wählenden Zeitdauer ("Duration of pumping, Pumpdauer") wird zu der jeweils angesteuerten Phase das zugehörige Lösungsmittel eingepumpt und zwar jeweils an den beiden Enden eines Rotors, so dass die Pumpen entgegengesetzt angeordnet sind. Die Probe bzw. das Substanzgemisch wird vorzugsweise intermediär in den Rotor eingespeist. In einer weiteren Ausführungsform können zwei oder mehrere Rotoren gekoppelt werden. Ein prinzipieller Zyklus stellt sich in Figur 1 abgebildet dar.

Die Rotorkammern werden vorzugsweise im Verhältnis 50/50 (Volumen %) mit einer oberen und einer unteren flüssigen Phase befüllt. Das Substanzgemisch wird mittels Pumpen kontinuierlich zwischen die beiden rotierenden Trennsäulen mit einer definierten Flussrate eingespeist und die Trennung erfolgt zyklisch und zeitversetzt, wobei in einem ersten Schritt die obere Phase als mobile Phase dient ("Ascending" ) und in einem zweiten Schritt die untere Phase als mobile Phase dient ("Descending" ).

Entsprechend der Konzentration und der unterschiedlichen Verteilungskoeffizienten der Substanzen im Substanzgemisch erfolgt die Auftrennung in zwei Produktströme, siehe Figur 1.

Geeignete Geräte und Apparaturen können von Armen Technologies (Frankreich) unter dem Namen "True Moving Bed CPC" erhalten werden.

Im Stand der Technik sind zu sCPC die Aufreinigung von Modellsubstanzen und binären Gemischen beschrieben (Göll, Johannes, Andreas Frey, and Mirjana Minceva. "Study of the Separation limits of continuous solid Support free liquid-liquid chromatography: Separation of capsaicin and dihydrocapsaicin by centrifugal partition chromatography." Journal of Chromatography A 1284 (2013) : 59-68; Hopmann E, Göll J, Minceva M. Sequential centrifugal partition chromatography: A new continuous Chromatographie technology. Chem. Eng. Technol. 2012; 35 (1) : 72-82 ; hier: binäres Gemisch aus Capsaicin und Dihydrocapsaicin), jedoch nicht Verfahren zur Trennung und/oder Reinigung von Naturstoffen aus Pflanzenextrakten und zwar zur Herstellung von Fraktionen und Reinstoffen.

In "Study of separation limits of continuous solid support free liquid-liquid chromatography: Separation and dihydrocapsaicin by centrifugal partition chromatography", Journal of chromatography A, Elsevier, Amsterdam, NL, Bd. 1284, 8. Februar 2013 (2013-02-08), Seiten 59-68, XP029000702, ISSN: 0021-9673, DOI: 10.1016/J.Chroma.2013.01.116 (Goll Johannes et al.) wird ein Feed-Gemisch aus Capsaicin und Dihydrocapsaicin ausgewählt, dessen Molekülstruktur sich nur in einer Doppelbindung unterscheidet. Es wird eine vollständige Trennung von Capsaicin und Dihydrocapsaicin mit dem Lösungsmittelsystem Heptan/Ethylacetat/Methanol/Wasser:l/l/l/l (v/v/v/v) beschrieben.

Weiterhin wird in "Extraction of cocoa proanthocyanidis and the fractionation by sequential centrifugal partition chromatography and gel permeation chromatography", Analytical and bioanalytical chemistry, Springer, DE, Bd. 408, Nr. 1, 18. Juni 2016 (2016-06-18), Seiten 5905-5914, XP036013845, ISSN: 1618-2642, DOI: 10.1007/500216-9705-7 (Pedan Vasilisa et al.) offenbart einen aktuellen Überblick über die Analyseverfahren, wobei der Schwerpunkt auf der Entwicklung einer schonenden Extraktionstechnik liegt. In dieser Arbeit wurden Kakaobohnen auf eine durchschnittliche Partikelgröße von < 100 µm fein gemahlen, bei 20 °C mit n-Hexan entfettet und bei 50 °C dreimal mit 50 % wässrigem Aceton extrahiert. Die Bestimmung des Gesamtphenolgehalts erfolgte mit dem Folin-Ciocalteu-Assay, die Konzentration der einzelnen Polyphenole wurde durch Elektrospray-Ionisations-Hochleistungsflüssigkeitschromatographie-Massenspektrometrie (ESI-HPLC/MS) analysiert. Die Fraktionen der bioaktiven Verbindungen wurden durch eine Kombination aus sequentieller Zentrifugalpartitionschromatographie (SCPC) und Gelpermeationssäulenchromatographie mit Sephadex LH-20 getrennt. Für die SCPC wurde ein zweiphasiges Lösungsmittelsystem, bestehend aus Ethylacetat/n-Butanol/Wasser (4:1:5, v/v/v), erfolgreich für die Trennung von Theobromin, Koffein und Vertretern der beiden wichtigsten phenolischen Verbindungsklassen Flavan-3-ole und Flavonole eingesetzt. Die Gelpermeationschromatographie auf Sephadex LH-20 unter Verwendung einer schrittweisen Elutionssequenz mit wässrigem Aceton hat gezeigt, dass einzelne Flavan-3-ole effektiv getrennt werden können. Eine Trennung wurde für (-)-Epicatechin, Proanthocyanidin-Dimer B2, Trimer C1 und Tetramer-Cinnamtannin A2 erzielt. Die Reinheit der Alkaloide und phenolischen Verbindungen wurde durch HPLC-Analyse bestimmt und ihre chemische Identität durch Massenspektrometrie bestätigt.

EP 3 061 510 A1 beschreibt ein Verfahren zur Trennung und/oder Reinigung von Cannabinoiden aus einem Cannabis-Extrakt, welches mindestens einen Flüssig-flüssig-Verteilungschromatographieschritt enthält und wobei ein Lösungsmittel aus Heptan ausgewählt ist, das durch Zentrifugalkraft stationär gehalten wird und eine zweite nichtmischbare flüssige Phase enthält.

In "Preparative Isolation of Cannabinoids from Cannabis sativa by Centrifugal Partition Chromatography", Journal of Liquid Chromatography & Related Technologies, Bd. 27, Nr. 15, 11. Januar 2004 (2004-01-11), Seiten 2421-2439, XPo55202081, ISSN: 1082-6076, DOI: 10.1081/JLC-200028170 wird ein Verfahren zur Isolierung von Cannabinoiden mittels CPC offenbart, in welchem ein zweiphasiges System bestehend aus Hexan/Methanol/Wasser oder Hexan/Aceton/Acetonitril verwendet wird.

Daher betrifft die Erfindung ein Verfahren zur Trennung und/oder Reinigung von einer oder mehr Cannabinoiden aus einem Cannabispflanzenextrakt, wobei das Verfahren mindestens einen Flüssig-flüssig-Verteilungschromatographieschritt an dem Cannabispflanzenextrakt umfasst, um das eine oder mehr Cannabinoid zu trennen und/oder zu reinigen, wobei beim Durchführen der mindestens einen Flüssig-flüssig-Verteilungschromatographie ein kontinuierlicher Wechsel der stationären Phase zur mobilen Phase und umgekehrt erfolgt (sCPC), wobei eine oder mehrere Fraktionen abgetrennt wird/werden, um das eine oder mehr Cannabinoide zu trennen und/oder zu reinigen.

Daher betrifft die Erfindung ein Verfahren zur Trennung und/oder Reinigung von einer oder mehr Cannabinoiden aus einem Cannabispflanzenextrakt , mindestens einen Flüssig-flüssig-Verteilungschromatographieschritt am dem Cannabinoidpflanzenextrakt umfasst, um das eine oder mehr Cannabinoide zu trennen und/oder zu reinigen, wobei beim Durchführen der mindestens eine Flüssig-flüssig-Verteilungschromatographie ein kontinuierlicher Wechsel der stationären Phase zur mobilen Phase und umgekehrt erfolgt (sCPC), wobei mindestens ein Reinstoff abgetrennt wird, um das eine oder mehr Cannabinoide zu trennen und/oder zu reinigen.

Im Rahmen dieser Erfindung wird unter einem "Pflanzenextrakt" ein Vielkomponentengemisch aus Naturstoffen verstanden, welches mehr als zwei Naturstoffe, insbesondere mehr als 10 oder 100 Naturstoffe, insbesondere mehr als 200, 300, 500 oder 1.000 Naturstoffe enthält. Pflanzenextrakte können beispielsweise mittels Extraktion, Perkolation oder Mazeration aus Pflanzenmaterialien gewonnen werden. Als Extraktionsmittel können Lösungsmittel, wie Wasser, C1-C5-Alkohole, Ethanol, oder andere Lösungsmittel mit ausreichender Polarität eingesetzt werden. Eine übliche Extraktion ist beispielsweise ein Gemisch aus Wasser / Ethanol (50:50, 70:30, 30:70) bei 70 Grad Celsius. Weiterhin sind solche Pflanzenextrakte erfindungsgemäß bevorzugt, die weniger als 50 Gewichts-% oder 40 Gewichts-% Fette oder Lipide aufweisen, insbesondere weniger als 20 Gewichts-% oder 10 Gewichts-% Fette oder Lipide, da Fette zu einer Beeinträchtigung der Trennleistung im sCPC-Verfahren führen können.

Nachstehende Gattungen sind Beispiele für Pflanzenextrakte:
*Equiseti, Juglandis, Millefolli, Quercus, Taraxaci, Althaeae, Matricariae, Centaurium, Levisticum, Rosmarinus, Angelica, Artemisia, Astragalus, Leonurus, Salvia, Saposhnikovia, Scutellaria, Siegesbeckia, Armoracia, Capsicum, Cistus, Echinacea, Galphimia, Hedera, Melia, Olea, Pelargonium, Phytolacca, Primula, Salix, Thymus, Vitex, Vitis, Rumicis, Verbena, Sambucus, Gentiana, Cannabis, Silybum.*

Nachstehende Arten sind Beispiele für die Pflanzenextrakte:
*Equiseti herba* (Schachtelhalmkraut), *Juglandis folium* (Walnussblätter), *Millefolli herba* (Schafgarbenkraut), *Quercus cortex* (Eichenrinde), *Taraxaci herba* (Löwenzahnkraut),
*Althaeαe radix* (Eibischwurzel) und *Matricariae flos* (bzw. *Flos chamomillae* (Kamilleblüten) ), *Centaurium erythraea* (Tausendgüldenkraut), *Levisticum officinale* (Liebstöckel), *Rosmarinus officinalis* (Rosmarin), *Angelica dahurica (Sibirische Engelwurz, PinYin Name: Baizhi), Angelica sinensis (Chinesische Angelika, PinYin Name: Danggui*) *, Artemisia scoparia (Besen-Beifuß, PinYin Name: Yinchen), Astragalus membranaceus* (var . *Mongolicus) (Traganthwurzel, Chin .: Huang- Qi), Leonurus japonicus* (Löwenohr, *Chin.: T'uei), Salvia miltiorrhiza (Rotwurzel-Salbei, Chin.: Danshen), Saposhnikovia divaricata (Siler, PinYin Name: Fangfeng*)*, Scutellaria baicalensis (Baikal-Helmkraut, Banzhilian), Siegesbeckia pubescens (Himmlisches Kraut, PinYin Name: Xixianciao), Armoracia rusticana (Meerrettich)* , *Capsicum sp. (Paprika), Cistus incanus (Zistrose), Echinacea angustifolia (Sonnenhut), Echinacea purpurea (Sonnenhut), Galphimia glauca, Hedera helix (Efeu), Melia toosendan (Chinesische Holunderfruchte, Chin.: Chuan Lian Zi) , Olea europaea (Olive), Pelargonium sp. (Pelagornien), Phytolacca americana (Kermesbeeren), Primula veris (Schlüsselblume), Salix sp. (Weide), Thymus L. (Thymian), Vitex agnus castus (Mönchspfeffer), Vitis vinifera (Edle Weinrebe), Rumicis herba (Sorrel herb), Verbena officinalis (Eisenkraut), Sambucus nigra (Schwarzer Holunder), Gentiana lutea (Gelber Enzian), Cannabis sativa (Hanf), Silybum marianum (Mariendistel),* wobei *Cannabis sativa* (Hanf) das Pflanzenextrakt gemäß der Erfindung ist.

Pflanzenextrakte sind besonders reich an heilbringenden Naturstoffen und sind als Arznei(mittel)pflanzen beschrieben, wie z.B. in den Produkten auf der Basis von Pflanzenextrakten der Bionorica SE (z.B. Bronchipret^{®}, Imupret^{®}, Sinupret^{®}) . Ferner weisen solche Pflanzen übliche charakteristische Stoffklassen an Naturstoffen auf, wie Sekundärmetaboliten, wie Flavonoide, Polyphenole, etc. Solche Arzneipflanzen dienen zum Bereitstellen von Arzneimitteln.

In einer bevorzugten Ausfiihrungsform wird der Pflanzenextrakt aus einem ersten Lösungsmittel, wie Alkohole, Ethanol, Wasser, Kohlenwasserstoffe, Heptan oder Mischungen davon gewonnen, und die löslichen Anteile in dem erfindungsgemäßen Verfahren eingesetzt (nach-, vorstehend: Substanzgemisch).

Zum erfindungsgemäßen Verfahren zur Trennung und/oder Reinigung von Naturstoffen aus Pflanzenextrakten mittels sCPC werden die kritischen Prozessparameter, nämlich Flussrate (flow rate) der oberen Phase, Flussrate der unteren Phase, Substanzgemisch-Flussrate, Zykluszeit "Descending" und Zykluszeit "Ascending" vorzugsweise gemäß folgenden Schritte durchgeführt:
- Festlegen einer definierten Konzentration des Substanzgemisches im ausgewählten Lösungsmittelsystem;
- Festlegen der maximalen Flussrate für die untere und obere Phase bei der kein unkontrolliertes Phasenbluten der flüssigen Phasen stattfindet;
- Festlegen der maximalen Substanzgemisch-Flussrate;
- Anpassen der Zykluszeit des Descending und / oder Ascending Modus um den Schnittpunkt der Trennung im Chromatogramm beliebig zwischen unterschiedlichen Substanzen zu verschieben, vorzugweise werden kurze Schaltzeiten verwendet um ein unkontrolliertes Phasenbluten und Austreten von nicht aufgetrennten Substanzen zu vermeiden. Dieses Vorgehen erlaubt die gezielte An- und Abreicherung als auch Abtrennung von Fraktionen oder von Reinstoffen, wie zum Beispiel nicht abschließend Cannabidiol, Tetrahydrocannabinol , Casticin (nicht erfindungsgemäß), Gentiopikrosid (nicht erfindungsgemäß), Agnusid (nicht erfindungsgemäß), Chamazulen (nicht erfindungsgemäß), Primulasaponin 1 (nicht erfindungsgemäß), u.a..

Beispielsweise können in den Fraktionen solche Stoffklassen von Pflanzenextrakten, wie Alkaloide, Bitterstoffe, Anthocyanine, Anthrachinone, Coumarine, Flavonoide, Glucosinolate, Lactone, Lignane, Lipide, Cannabinoide, Phenole, Polyphenole, Saponine, Terpene, Xanthone an- oder abgereichert werden, die abgetrennt werden können Besonders vorteilhaft können solche Fraktionen und Reinstoffe in hoher Reinheit und Ausbeute erhalten werden.

Zum Einsatz kommende Lösungsmittel können bei der Flüssig- Flüssig Verteilungschromatographie beispielsweise Skalicka- Wozniak K, Garrard I, A comprehensive Classification of solvent Systems used for natural product purifications in countercurrent and centrifugal partition chromatography, Nat Prod Rep . 2015 Nov; 32 (11) : 1556-61 entnommen werden.

Erfindungsgemäße Beispiele für geeignete Lösungsmittel aus denen zweiphasige Lösungsmittelsysteme (mobile Phase /stationäre Phase) für Pflanzenextrakte bereitgestellt werden können, sind:
a. ) Kohlenwasserstoffe wie n-Hexan, Cyclohexan, Isohexan,
   Heptan, Isooctan;
b. ) Ether wie t-Butylmethylether, Petrolether, Diethylether;
c. ) Halogenierte Lösungsmittel wie Chloroform, Dichlormethan, Benzotrifluorid, Dichlorethan, Tetrachlormethan, Trichlorethan;
d. ) Wasser-Lösliche Alkhole wie Butanol, Methanol, Ethanol, Isopropanol;
e. ) Wasser-Lösliche Ester wie Ethylacetat, Isopropylacetat;
f. ) Acetonitril, Toluol.

Aus diesen vorgenannten Lösungsmitteln lassen sich geeignete zweiphasige Lösungsmittelsysteme herstellen, vorzugsweise solche wie, insbesondere zumindest enthaltend:
n-Heptan und/oder Acetonitril, n-Heptan/Acetonitril
n-Heptan/Ethylacetat /Acetonitril;
n-Heptan/Ethylacetat /t-Butylmethylether/ Acetonitril;
n-Heptan/Ethylacetat /Methanol /Wasser;
n-Heptan/Ethanol /Wasser.

Daher betrifft die Erfindung ebenfalls ein Verfahren zur Trennung und/oder Reinigung von einer oder mehr Cannabinoiden aus einem Cannabispflanzenextrakt, enthaltend mindestens einen Flüssig-flüssig-Verteilungschromatographieschritt, wobei ein kontinuierlicher Wechsel der stationären Phase zur mobilen Phase und umgekehrt erfolgt, und die stationäre Phase als auch mobile Phase aus mindestens zwei Lösungsmitteln ausgewählt sind aus Kohlenwasserstoffe mit 5 - 8
Kohlenstoffatomen, insbesondere n-Heptan, Acetonitril, Ethylacetat, t-Butylmethylether, Alkohole, insbesondere Methanol, Wasser, Ethanol, n-Heptan und / oder Acetonitril, so dass ein zwei-Phasen-System erhalten werden kann.

Ganz besonders bevorzugt sind jedoch n-Heptan und / oder Acetonitril zur Auftrennung der Pflanzenextrakte mittels dem erfindungsgemäßen sCPC-Verfahren. Weiterhin ist zur optimalen Trennung der Pflanzenextrakte bevorzugt, dass der Rotor auf eine Rotation zur Durchführung des erfindungsgemäßen Verfahrens auf mehr als 2.000 U/min (rpm), insbesondere 2.500 U/min (rpm) und mehr beschleunigt wird.

Nachfolgende Beispiele und Figuren dienen ausschließlich zur Erläuterung der Erfindung, ohne die Erfindung auf diese Beispiele zu beschränken.

### Beispiel 1 :

### Darstellung eines Equipments:

### Ausrüstung:

Kontinuierlicher Flüssig-Flüssig Chromatograph True-Moving-Bed (TMB) 500 System, mit 2x250 mL Rotor, Drehzahl 100-3000 rpm, bis 100 mL/min Flussrate, 100 bar Maximaldruck von Armen Instruments (Saint-Ave, Frankreich) oder 12 L Produktionsanlage mit 2x6L Rotor.

### Zusatzausrüstung:

2xEluenten-Pumpe, bis 100 mL/min Flussrate; 2xProbenaufgabe- Pumpe, bis 50 mL/min Flussrate; 2xDioden-Array-Detektor Ecom, 4 Wellenlängen, 200-600 nm Detektionsbereich, 6 kg; 2xFraktionensammler LS-5600, inkl. Rack-Set aus Edelstahl, 20 kg

### Funktionsweise:

Die Probe bzw. das Substanzgemisch wird kontinuierlich, phasenweise auf beide Rotoren injiziert und die Probenkomponenten nach Polarität und gemäß ihrer flüssig- flüssig-Verteilungskoeffizienten K mit einem geeigneten Zweiphasigen Lösungsmittelsystem getrennt. Nach Anfahren der Anlage stellt sich ein stationärer, steady-state Zustand ein. An einem Rotor kann anschließend mittels eines Fraktionensammlers Produktseite A entnommen werden. Abwechselnd kann am zweiten Rotor mit einem weiteren Fraktionensammler Produktseite B entnommen werden kann. Die Detektoren dienen zur Überwachung der Trennung.

### Beispiel 2 :

### Nutzhanfextrakt unter Abreicherung von THC

Gewinnung von Nutzhanfextrakt THC-befreit: Extraktion Cannabis herbe mittels CO2, Decarboxylierung bei 120°C im Vakuum,
Auflösen in n-Heptan/Ethanol/Wasser,
Methode:
   Parameter:
   Rotation: 2600 rpm
Ascending Modus:
   Flow rate Elution: 30 ml/min
   Flow rate Injection: 10 ml/min / 5 ml/min
   Pumpdauer: 90 sec.
Descending Modus:
   Flow rate Elution: 30 ml/min
   Flow rate Injection: 10 ml/min / 5 ml/min
   Pumpdauer: 99 sec.
   Flow rate = Flussrate

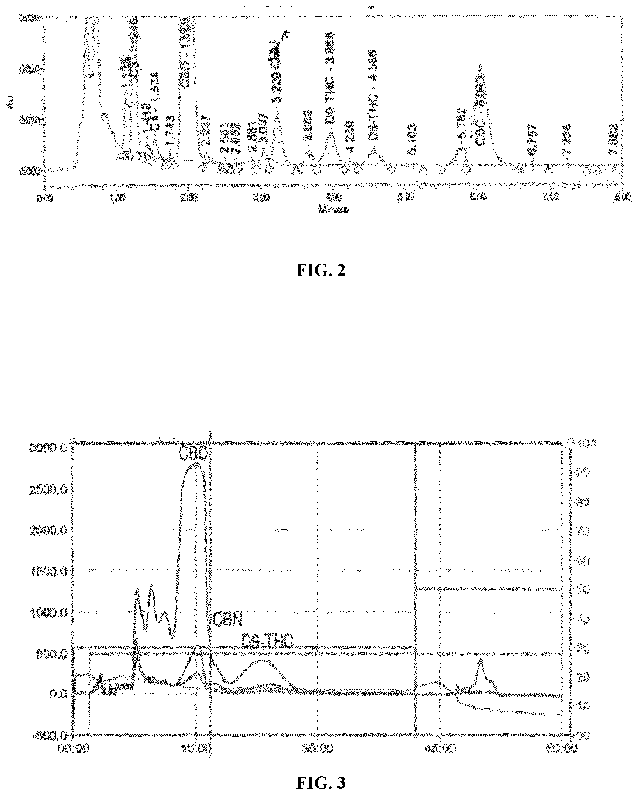

Das HPLC-Chromatogramm Ausgangsmaterials wird in FIG. 2 gezeigt.

Gehaltsanalyse: CBD: 51,54%, D9-THC: 1,21%, CBN: 1,38% (siehe FIG.2) Ergebnisse der Batch Verteilungschromotagraphie (CPC) werden in FIG. 3 gezeigt.

Trennung von 3,05 g Ausgangsprodukt (50g/L) in 30 min in Batchbetrieb mit n-Heptan/Ethanol/Wasser (5:4:1.4) - die vertikale Linie bei 17:00 beschreibt die Trennlinie bei der späteren kontinuierlichen Verteilungschromatographie.

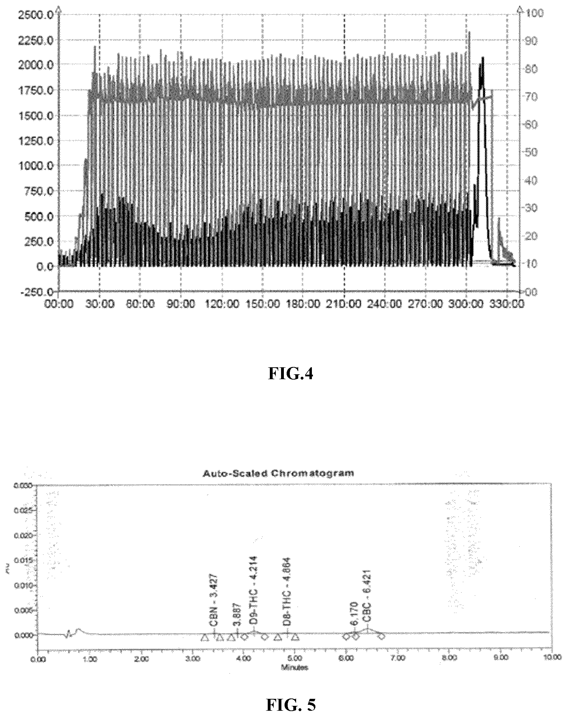

Ergebnisse der kontinuierlichen Verteilungschromatographie (sCPC) werden in FIG. 4. gezeigt.

Trennung im kontinuierlichen Betrieb (50g/L) mit der SCPC mit n-Heptan/Ethanol/Wasser (5:4:1.4)
Grau: UV-Signal Descending Seite, Schwarz: UV-Signal Ascending Seite

Das HPLC-Chromatogramm der Ascending Seite wird in FIG. 5 gezeigt.

Ergebnisse der Gehaltsanalyse werden in FIG. 5 gezeigt: CBD : 0,00%, D9-THC: 2,63%, CBN: 2,84% .

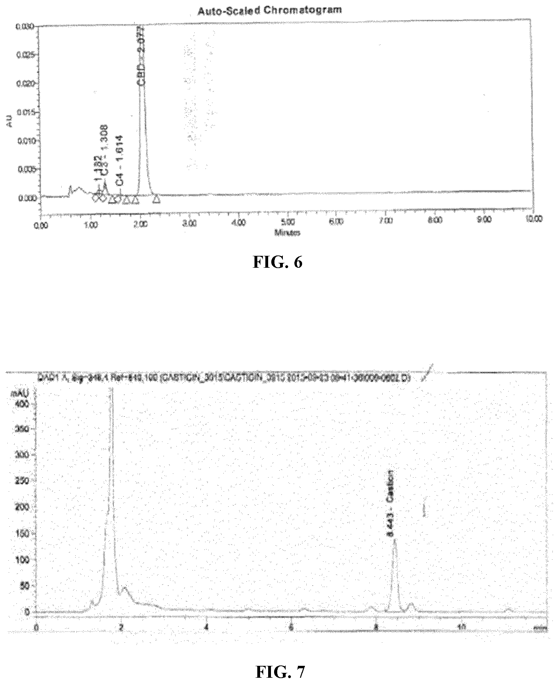

In FIG. 6 wird ein HPLC-Chromatogramm der Descending Seite gezeigt.

Gehaltsanalyse: CBD: 96,22%, D9-THC: 0,00%, CBN: 0,00%

### Tabelle 1

### Beispiel 3 (nicht erfindungsgemäß):

### Aufreinigung von Casticin

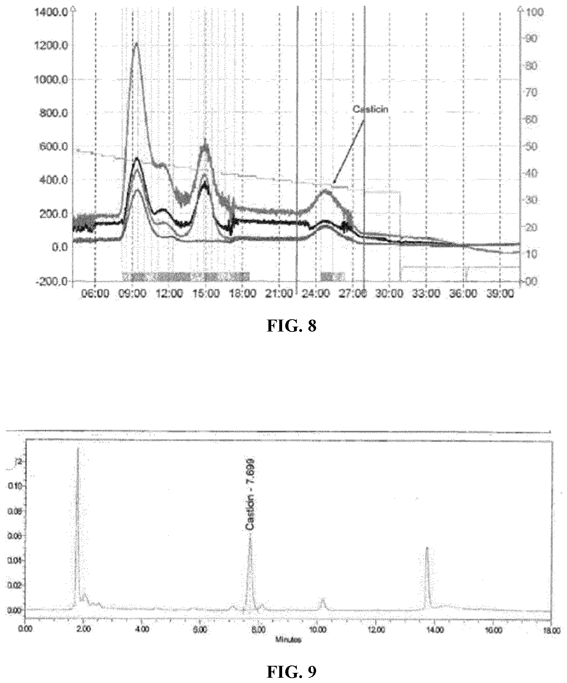

Parameter:
   Rotation: 2600 rpm
Ascending Modus:
   Flow rate Elution 15 ml/min
   Flow rate Injection: 5 ml/min
   Pumpdauer : 30 sec.
Descending Modus:
   Flow rate Elution: 15 ml/min
   Flow rate Injection: 5 ml/min
   Pumpdauer : 60 sec.
Das HPLC Chromatogramm Ausgangsprodukt Essi Agni Casti wird in FIG. 7 gezeigt. Lösungsmittelsystem: n-Heptan / Ethanol / Wasser (5 : 4: 1)
   (v / v / v)
Gehalt an Casticin im Trockenextrakt: 0,145% (m/m)
Ergebnisse der Batch Verteilungschromatographie (CPC) werden in FIG. 8. gezeigt.

Trennung von 1 g Essi Agni Casti im Batchbetrieb mit n- Heptan/Ethylacetat /Ethanol/Wasser (7:10:7:10) - die vertikalen Linien bei 22:00 und 28:00 beschreiben die Trennungen bei der späteren kontinuierlichen Verteilungschromatographie.

Ergebnisse des True Moving Bed Aufreinigungsschritt 1 zur Voranreicherung werden in FIG. 9. gezeigt.

Gehalt an Casticin: 60,3% (m/m) in FIG. 9.

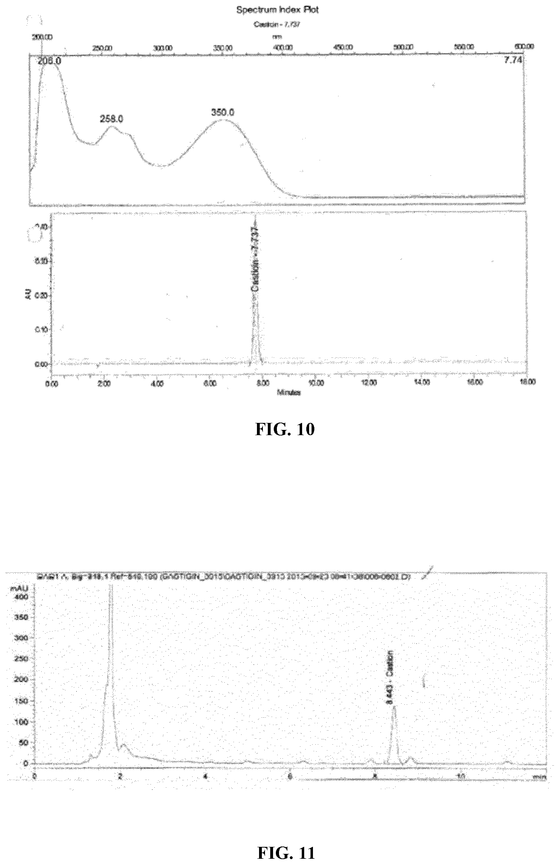

Ergebnisse des True Moving Bed Aufreinigungsschritt 2 zur Voranreicherung werden in FIG. 10. gezeigt.

Das DAD Spektrum (oben) und HPLC Chromatogramm (unten) aufgereinigtes Casticin nach TMB werden in FIG. 10 gezeigt..

Gehalt an Casticin: 98,3% (m/m) in FIG. 10.

Das HPLC-Chromatogramm des Ausgangsproduktes Essi Agni Casti wird in FIG. 11 gezeigt.

Gehalt an Casticin im Trockenextrakt: 0,145% (m/m) in FIG. 11.

Lösungsmittelsystem: n-Heptan / Ethylacetat / Ethanol / Wasser (7 : 10 : 7: 10) (v / v / v / v) in FIG. 11.

### Beispiel 4 (nicht erfindungsgemäß):

### Aufreinigung Chamazulen

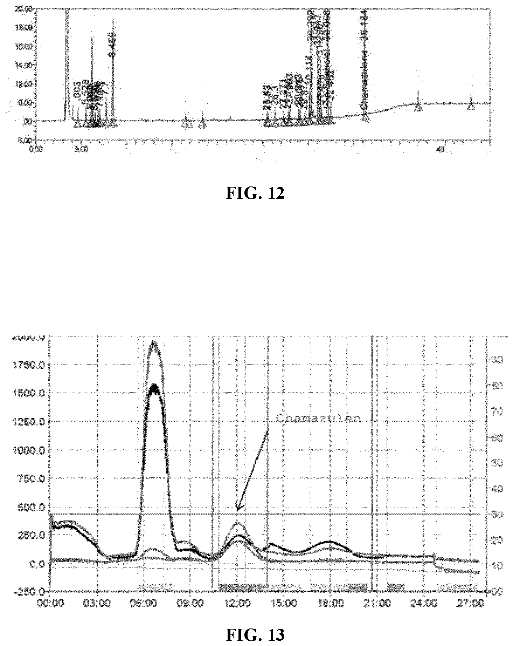

Das GC-Chromatogramm des Ausgangsproduktes Oleum Chamomillae wird in FIG. 12. gezeigt.

Gehalt an Chamazulen im Ätherischen Öl: 15,8% (m/m) in FIG. 12.

Die Batch Verteilungschromatographie (CPC) wird in FIG. 13 gezeigt.

Trennung von 0,5 g Oleum Chamomillae im Batchbetrieb mit n-Heptan/TBME/Acetonitril (4:2:4) - die blauen Linien beschreiben die Trennungen bei der späteren kontinuierlichen Verteilungschromatographie

### 1. TMB Schritt

Parameter :
   Rotation: 2600 rpm
Ascending Modus:
   Flow rate Elution: 15 ml/min
   Flow rate Injection: 5 ml/min
   Pumpdauer : 90 sec.
Descending Modus:
   Flow rate Elution: 15 ml/min
   Flow rate Injection: 5 ml/min
   Pumpdauer: 75 sec.
   Lösungsmittelsystem: n-Heptan/TBME/Acetonitril (4 : 2 : 4) (v / v / v )

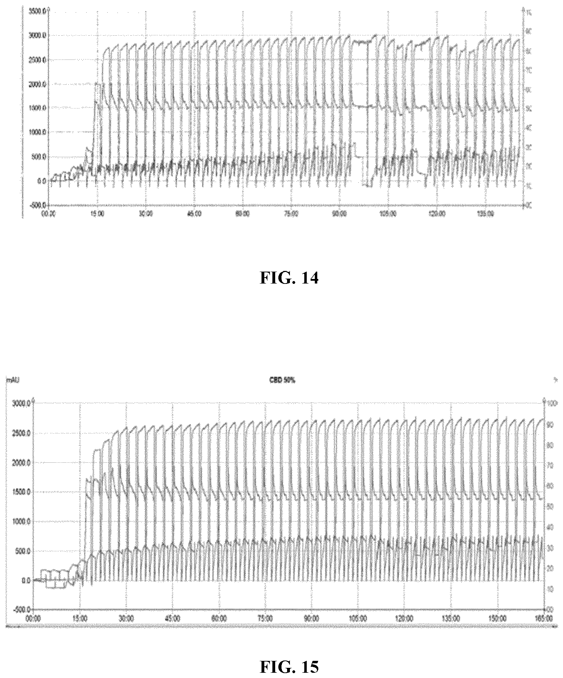

Ergebnisse des 1.TMB Schritt Kontinuierliche Verteilungschromatographie (SCPC) werden in FIG. 14. gezeigt.

Trennung von Oleum Chamomillae (25 g/L) im kontinuierlichen Betrieb mit der SCPC mit n-Heptan/Ethylacetat /Ethanol/Wasser (7:10:7:10) als Aufreinigungsschritt 1
Gehalt an Chamazulen nach Voranreicherung TMBl: 70,3% (m/m) in FIG. 14.

### 2. TMB Schritt

Parameter :
   Rotation : 2600 rpm
Ascending Modus:
   Flow rate Elution: 15 ml/min
   Flow rate Injection: 5 ml/min
   Pumpdauer : 30 sec.
Descending Modus:
   Flow rate Elution: 15 ml/min
   Flow rate Injection: 5 ml/min
   Pumpdauer: 60 sec.
Lösungsmittelsystem: n-Heptan/TBME/Acetonitril (4 : 2 : 4) (v / v / v )

Ergebnisse des 2. TMB Schritt Kontinuierliche Verteilungschromatographie (SCPC) werden in FIG. 15. gezeigt.
Trennung des Materials aus TMB-Schritt 1 (25 g/L) im
kontinuierlichen Betrieb mit der SCPC mit n-
Heptan/TBME/Acetonitril (4 : 2 : 4) als Aufreinigungsschritt 2.

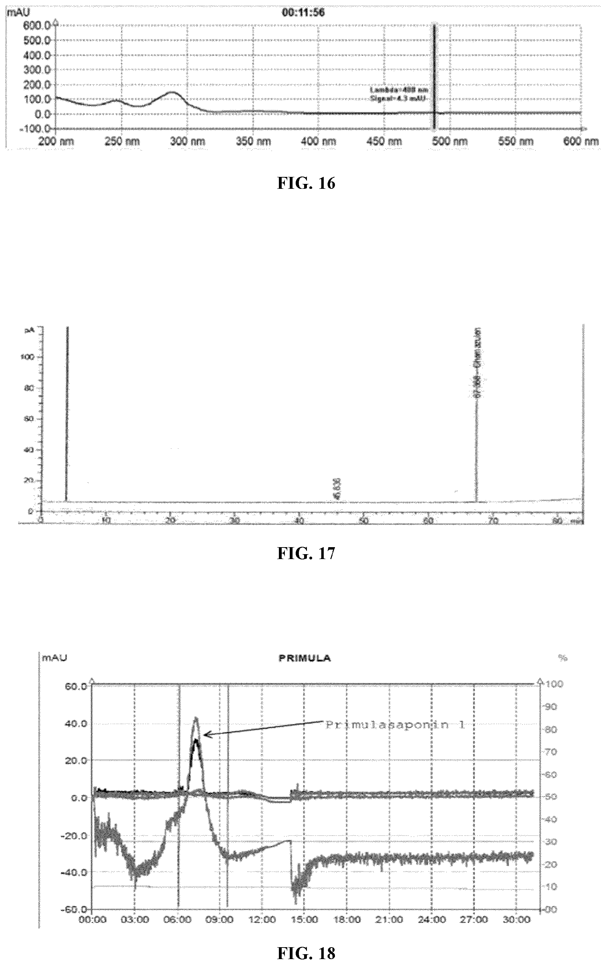

Das DAD Spektrum (oben) und GC Chromatogramm (unten) aufgereinigtes Chamazulen nach TMB Aufreinigungsschritt 2 werden in FIG. 16. gezeigt.

Gehalt an Chamazulen: 99,0% (m/m) siehe in FIG. 17.

### Beispiel 5 (nicht erfindungsgemäß):

Aufreinigung Primulasaponine
Essi Primula veris
Gehalt an Primulasaponin 1 im Extrakt: -20% (m/m)
Die Batch Verteilungschromatographie (CPC) wird in FIG. 18. gezeigt.

Trennung von 0,1 g Essi Primula veris im Batchbetrieb mit n- Heptan/Ethylacetat /Wasser (6:5:4) - die blauen Linien beschreiben die Trennungen bei der späteren kontinuierlichen Verteilungschromatographie

### 1. TMB Schritt

Parameter:
   Rotation: 2600 rpm
Ascending Modus:
   Flow rate Elution: 15 ml/min
   Flow rate Injection 5 ml/min
   Pumpdauer : 90 sec.
Descending Modus:
   Flow rate Elution: 15 ml/min Flow rate Injection: 5 ml/min
   Pumpdauer: 75 sec.
   Lösungsmittelsystem: n-Heptan/Ethylacetat /Wasser (6:5:4) (v / v / v )

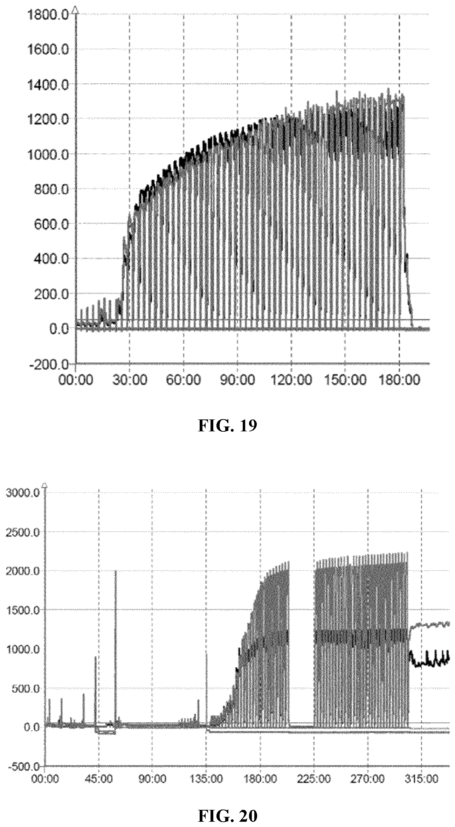

Ergebnisse der. 1 TMB Schritt Kontinuierlichen Verteilungschromatographie (SCPC) werden in FIG. 19. gezeigt.

Trennung von Essi Primula veris (5 g/L) im kontinuierlichen Betrieb mit der SCPC mit n-Heptan/Ethylacetat /Wasser (6:5:4) als Aufreinigungsschritt 1
Gehalt an Primulasaponin 1 nach Voranreicherung TMBl: 60,1% (m/m)

### 2. TMB Schritt

Parameter:
   Rotation: 2600 rpm
Ascending Modus:
   Flow rate Elution: 15 ml/min
   Flow rate Injection: 5 ml/min
   Pumpdauer: 30 sec.
Descending Modus:
   Flow rate Elution: 15 ml/min
   Flow rate Injection: 5 ml/min
   Pumpdauer: 60 sec.
Lösungsmittelsystem: n-Heptan/Ethylacetat /Wasser (6:5:4) (v / v / v )

Ergebnisse der 2.TMB Schritt Kontinuierliche Verteilungschromatographie (SCPC) werden in FIG. 20. gezeigt.

Trennung des Materials aus TMB-Schritt 1 (5 g/L) im
kontinuierlichen Betrieb mit der SCPC mit n- Heptan/Ethylacetat /Wasser (6:5:4) als Aufreinigungsschritt 2 (siehe FIG. 20).

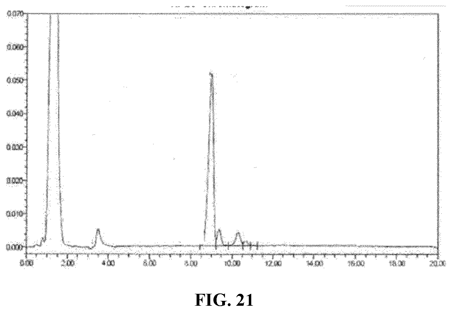

Das DAD Spektrum (oben) und HPLC Chromatogramm (unten) aufgereinigtes Primulasaponin 1 nach TMB Aufreinigungsschritt 2 werden in FIG. 21. gezeigt.

Gehalt an Primulasponin 1: 84,0% in FIG. 21.

## Patentansprüche

1. Verfahren zur Trennung und/oder Reinigung von einer oder mehr Cannabinoiden aus einem Cannabispflanzenextrakt, wobei das Verfahren einen Schritt Durchführen mindestens einer Flüssig-flüssig-Verteilungschromatographie an dem Cannabispflanzenextrakt umfasst, um das eine oder mehr Cannabinoid zu trennen und/oder zu reinigen, wobei beim Durchführen der mindestens einen Flüssig-flüssig-Verteilungschromatographie ein kontinuierlicher Wechsel der stationären Phase zur mobilen Phase und umgekehrt erfolgt, **dadurch gekennzeichnet, dass** eine oder mehrere Fraktionen abgetrennt wird/werden, um das eine oder mehr Cannabinoid zu trennen und/oder zu reinigen.

2. Verfahren zur Trennung und/oder Reinigung von einer oder mehr Cannabinoiden aus einem Cannabispflanzenextrakt, wobei das Verfahren einen Schritt Durchführen mindestens einer Flüssig-flüssig-Verteilungschromatographie an dem Cannabispflanzenextrakt umfasst, um das eine oder mehr Cannabinoid zu trennen und/oder zu reinigen, wobei beim Durchführen der mindestens einen Flüssig-flüssig-Verteilungschromatographie ein kontinuierlicher Wechsel der stationären Phase zur mobilen Phase und umgekehrt erfolgt, **dadurch gekennzeichnet, dass** mindestens ein Reinstoff abgetrennt wird, um das eine oder mehr Cannabinoid zu trennen und/oder zu reinigen.

3. Verfahren zur Trennung und/oder Reinigung von einer oder mehr Cannabinoiden aus einem Cannabispflanzenextrakt nach Anspruch 1 oder 2, **dadurch gekennzeichnet, dass** mindestens ein Rotor eingesetzt wird.

4. Verfahren zur Trennung und/oder Reinigung von einer oder mehr Cannabinoiden aus einem Cannabispflanzenextrakt nach Anspruch 3, **dadurch gekennzeichnet, dass** eine Rotation von mehr als 2.000 U/min. (rpm), insbesondere 2.500 U/min. (rpm) erfolgt.

5. Verfahren zur Trennung und/oder Reinigung von einer oder mehr Cannabinoiden aus einem Cannabispflanzenextrakt nach einem der vorhergehenden Ansprüche, **dadurch gekennzeichnet, dass** der Pflanzenextrakt mehr als zwei Naturstoffe enthält.

6. Verfahren zur Trennung und/oder Reinigung von einer oder mehr Cannabinoiden aus einem Cannabispflanzenextrakt nach einem der vorhergehenden Ansprüche, **dadurch gekennzeichnet, dass** der Pflanzenextrakt weniger als 40 Gewichts-% Fette/Lipide aufweist.

7. Verfahren zur Trennung und/oder Reinigung von einer oder mehr Cannabinoiden aus einem Cannabispflanzenextrakt nach einem der vorhergehenden Ansprüche, **dadurch gekennzeichnet, dass** der Pflanzenextrakt aus einem ersten Lösungsmittel, wie Alkohole, Ethanol, Wasser, Kohlenwasserstoffe, Heptan oder Mischungen davon gewonnen wird, und die löslichen Anteile eingesetzt werden.

8. Verfahren zur Trennung und/oder Reinigung von einer oder mehr Cannabinoiden aus einem Cannabispflanzenextrakt nach einem der vorhergehenden Ansprüche, **dadurch gekennzeichnet, dass** das eine oder mehr Cannabinoid ausgewählt ist aus Cannabidiol und Tetrahydrocannabinol.

9. Verfahren zur Trennung und/oder Reinigung von einer oder mehr Cannabinoiden aus einem Cannabispflanzenextrakt nach einem der vorhergehenden Ansprüche, **dadurch gekennzeichnet, dass** das eine oder mehr Cannabinoid Cannabidiol ist.

10. Verfahren zur Trennung und/oder Reinigung von einer oder mehr Cannabinoiden aus einem Cannabispflanzenextrakt nach einem der vorhergehenden Ansprüche, **dadurch gekennzeichnet, dass** das Pflanzenextrakt *Cannabis sativa* ist.

11. Verfahren zur Trennung und/oder Reinigung von einer oder mehr Cannabinoiden aus einem Cannabispflanzenextrakt nach Anspruch 10, **dadurch gekennzeichnet, dass** das Cannabis-sativa-Planzenextrakt ein Nutzhanfextrakt ist.

12. Verfahren zur Trennung und/oder Reinigung von einer oder mehr Cannabinoiden aus einem Cannabispflanzenextrakt nach einem der vorhergehenden Ansprüche, **dadurch gekennzeichnet, dass** die stationäre Phase und mobile Phase unabhängig aus mindestens zwei Lösungsmitteln ausgewählt sind aus Kohlenwasserstoffe mit 5 - 8 Kohlenstoffatomen, n-Heptan, Acetonitril, Ethylacetat, t-Butylmethylether, Alkohole, Methanol, Wasser, und Ethanol, so dass ein zwei-Phasen-System erhalten werden kann.

13. Verfahren zur Trennung und/oder Reinigung von einer oder mehr Cannabinoiden aus einem Cannabispflanzenextrakt nach Anspruch 12, **dadurch gekennzeichnet, dass** die Lösungsmittel zumindest enthaltend sind:
a.) n-Heptan und/oder Acetonitril,
b.) n-Heptan/Ethylacetat/Acetonitril;
c.) n-Heptan/Ethylacetat/t-Butylmethylether/Acetonitril;
d.) n-Heptan/Ethylacetat/Methanol/Wasser;
e.) n-Heptan/Ethanol/Wasser.

14. Verfahren zur Trennung und/oder Reinigung von einer oder mehr Cannabinoiden aus einem Cannabispflanzenextrakt nach Anspruch 13, **dadurch gekennzeichnet, dass** das Lösungsmittel n-Heptan/Ethanol/Wasser enthält.

## Claims

1. A method for separating and/or purifying one or more cannabinoids from a cannabis plant extract, the method comprising a step of performing at least one liquid-liquid partition chromatography on the cannabis plant extract to separate and/or purify the one or more cannabinoids, wherein in performing the at least one liquid-liquid partition chromatography a continuous change of stationary phase to mobile phase and vice versa takes place, **characterised in that** several fractions are separated to separate and/or purify the one or more cannabinoids.

2. A method for separating and/or purifying one or more cannabinoids from a cannabis plant extract, the method comprising a step of performing at least one liquid-liquid partition chromatography on the cannabis plant extract to separate and/or purify the one or more cannabinoids, wherein when performing the at least one liquid-liquid partition chromatography a continuous change of the stationary phase to the mobile phase and vice versa takes place, **characterised in that** at least one pure substance is separated to separate and/or purify the one or more cannabinoids.

3. The method for the separation and/or purification of one or more cannabinoids from a cannabis plant extract according to claim 1 or 2, **characterised in that** a rotor is used.

4. The method for the separation and/or purification of one or more cannabinoids from a cannabis plant extract according to claim 3, **characterised in that** a rotation of more than 2,000 rpm. (rpm), in particular 2,500 rpm. (rpm) takes place.

5. The method for separating and/or purifying one or more cannabinoids from a cannabis plant extract according to one of the preceding claims, **characterised in that** the plant extract contains more than two natural substances.

6. The method for the separation and/or purification of one or more cannabinoids from a cannabis plant extract according to any one of the preceding claims, **characterised in that** the plant extract comprises less than 40% by weight of fats/lipids.

7. The method for the separation and/or purification of one or more cannabinoids from a cannabis plant extract according to any one of the preceding claims, **characterised in that** the plant extract is obtained from a first solvent, such as alcohols, ethanol, water, hydrocarbons, heptane or mixtures thereof, and the soluble portions are used.

8. The method for the separation and/or purification of one or more cannabinoids from a cannabis plant extract according to any of the preceding claims, **characterised in that** the one or more cannabinoid is selected from cannabidiol and tetrahydrocannabinol.

9. The method for separating and/or purifying one or more cannabinoids from a cannabis plant extract according to any one of the preceding claims, **characterised in that** the one or more cannabinoid is cannabidiol.

10. The method for separating and/or purifying one or more cannabinoids from a cannabis plant extract according to any one of the preceding claims, **characterised in that** the plant extract is *Cannabis sativa.*

11. The method for separating and/or purifying one or more cannabinoids from a cannabis plant extract according to claim 10, **characterised in that** the *Cannabis sativa* plant extract is an industrial hemp extract.

12. The method for the separation and/or purification of one or more cannabinoids from a cannabis plant extract according to any of the preceding claims, **characterised in that** the stationary phase and mobile phase are independently selected from at least two solvents selected from hydrocarbons having 5 - 8 carbon atoms, n-heptane, acetonitrile, ethyl acetate, t-butyl methyl ether, alcohols, methanol, water, and ethanol, so that a two-phase system can be obtained.

13. The method for the separation and/or purification of one or more cannabinoids from a cannabis plant extract according to claim 12, **characterised in that** the solvents are at least comprising:
a.) n-heptane and/or acetonitrile,
b.) n-heptane/ethyl acetate/acetonitrile;
c.) n-heptane/ethyl acetate/t-butyl methyl ether/acetonitrile;
d.) n-heptane/ethyl acetate/methanol/water;
e.) n-heptane/ethanol/water.

14. The method for the separation and/or purification of one or more cannabinoids from a cannabis plant extract according to claim 13, **characterised in that** the solvent contains n-heptane/ethanol/water.

## Revendications

1. Procédé de séparation et/ou de purification d'un ou de plusieurs cannabinoïdes à partir d'un extrait des plantes de cannabis, ledit procédé comprenant une étape consistant à soumettre ledit extrait de plantes de cannabis à au moins une chromatographie de partage liquide/liquide, afin de séparer et/ou purifier ledit ou lesdits cannabinoïdes, l'au moins une chromatographie de partage liquide/liquide étant réalisée en effectuant de manière continue une alternance de la phase stationnaire vers la phase mobile ou inversement, **caractérisé en ce qu'**une ou plusieurs fraction(s) est/sont enlevée(s) par séparation afin de séparer et/ou purifier ledit ou lesdits cannabinoïdes.

2. Procédé de séparation et/ou de purification d'un ou de plusieurs cannabinoïdes à partir d'un extrait des plantes de cannabis, ledit procédé comprenant une étape consistant à soumettre ledit extrait de plantes de cannabis à au moins une chromatographie de partage liquide/liquide, afin de séparer et/ou purifier ledit ou lesdits cannabinoïdes, l'au moins une chromatographie de partage liquide/liquide étant réalisée en effectuant de manière continue une alternance de la phase stationnaire vers la phase mobile ou inversement, **caractérisé en ce qu'**au moins une substance pure est enlevée par séparation afin de séparer et/ou purifier ledit ou lesdits cannabinoïdes.

3. Procédé de séparation et/ou de purification d'un ou de plusieurs cannabinoïdes à partir d'un extrait des plantes de cannabis selon les revendications 1 ou 2, **caractérisé en ce que** l'on met en oeuvre au moins un rotor.

4. Procédé de séparation et/ou de purification d'un ou de plusieurs cannabinoïdes à partir d'un extrait des plantes de cannabis selon la revendication 3, **caractérisé en ce que** l'on met on oeuvre une rotation dépassant 2 000 tours/min. (rpm), notamment 2 500 tours/minute (rpm).

5. Procédé de séparation et/ou de purification d'un ou de plusieurs cannabinoïdes à partir d'un extrait des plantes de cannabis selon l'une des revendications précédentes, **caractérisé en ce que** l'extrait de plantes contient plus de deux substances naturelles.

6. Procédé de séparation et/ou de purification d'un ou de plusieurs cannabinoïdes à partir d'un extrait des plantes de cannabis selon l'une des revendications précédentes, **caractérisé en ce que** l'extrait de plantes comporte moins de 40 % en poids de graisses/lipides.

7. Procédé de séparation et/ou de purification d'un ou de plusieurs cannabinoïdes à partir d'un extrait des plantes de cannabis selon l'une des revendications précédentes, **caractérisé en ce que** l'extrait de plantes est obtenu à partir d'un premier solvant, pouvant s'agir d'alcools, d'éthanol, d'eau, d'hydrocarbures, d'heptane ou de leurs mélanges, et les parties solubles sont mises en œuvre.

8. Procédé de séparation et/ou de purification d'un ou de plusieurs cannabinoïdes à partir d'un extrait des plantes de cannabis selon l'une des revendications précédentes, **caractérisé en ce que** ledit un ou lesdits cannabinoïdes est/sont sélectionné(s) parmi le cannabidiol et le tétrahydrocannabinol.

9. Procédé de séparation et/ou de purification d'un ou de plusieurs cannabinoïdes à partir d'un extrait des plantes de cannabis selon l'une des revendications précédentes, **caractérisé en ce que** ledit un ou lesdits cannabinoïdes correspond(ent) à du cannabidiol.

10. Procédé de séparation et/ou de purification d'un ou de plusieurs cannabinoïdes à partir d'un extrait des plantes de cannabis selon l'une des revendications précédentes, **caractérisé en ce que** l'extrait de plantes correspond à du *Cannabis sativa.*

11. Procédé de séparation et/ou de purification d'un ou de plusieurs cannabinoïdes à partir d'un extrait des plantes de cannabis selon l'une des revendications précédentes, **caractérisé en ce que** l'extrait de plantes de Cannabis sativa est un extrait de chanvre agricole.

12. Procédé de séparation et/ou de purification d'un ou de plusieurs cannabinoïdes à partir d'un extrait des plantes de cannabis selon l'une des revendications précédentes, **caractérisé en ce que** la phase stationnaire et la phase mobile sont choisies, de manière indépendante, parmi au moins deux solvants, à savoir parmi les hydrocarbures comportant 5 à 8 atomes de carbone, le n-heptane, l'acétonitrile, l'acétate d'éthyle, le t-butylméthyléther, les alcools, le méthanol, l'eau et l'éthanol, faisant en sorte que l'on puisse obtenir un système biphasique.

13. Procédé de séparation et/ou de purification d'un ou de plusieurs cannabinoïdes à partir d'un extrait des plantes de cannabis selon la revendication 12, caractérisé en ce lesdits solvants correspondent à :
a.) du n-heptane et/ou de l'acétonitrile,
b.) du n-heptane / acétate d'éthyle / acétonitrile ;
c.) du n-heptane / acétate d'éthyle / t-butylméthyléther / acétonitrile ;
d.) du n-heptane / acétate d'éthyle / méthanol / eau ;
e.) du n-heptane / éthanol / eau.

14. Procédé de séparation et/ou de purification d'un ou de plusieurs cannabinoïdes à partir d'un extrait des plantes de cannabis selon la revendication 13, caractérisé en ce ledit solvant contient du n-heptane / éthanol / eau.
